# EUROPEAN PATENT SPECIFICATION

(11) **EP 3 939 661 B1**
(45) Date of publication and mention of the grant of the patent: **03.09.2025**
(21) Application number: 20770505.4
(22) Date of filing: 27.02.2020
(51) Int. Cl.: A61P 23/02, A61K 9/70, A61K 47/14, A61K 47/32, A61K 31/167, A61K 31/245, A61K 31/445

(54) **PATCH**
PFLASTER
TIMBRE TRANSDERMIQUE

(30) Priority: 14.03.2019 JP 2019047009
(43) Date of publication of application: 19.01.2022
(73) Proprietor: KANEKA CORPORATION, Osaka 530-8288 (JP)
(72) Inventor: OGINO, Hiroyuki, Takasago-shi, Hyogo 676-8688 (JP); AKAZAWA, Mitsuji, Takasago-shi, Hyogo 676-8688 (JP); GOTO, Masaoki, Takasago-shi, Hyogo 676-8688 (JP)
(74) Representative: Hoffmann Eitle
(86) International application number: PCT/JP2020/008173
(87) International publication number: WO 2020/184208

(56) References cited:
- WO-A1-2018/230687
- WO-A1-2019/142940
- JP-A- 2002 080 350
- JP-A- 2008 266 175

## Description

### TECHNICAL FIELD

The present invention relates to a patch comprising a local anesthetic agent.

### TECHNICAL FIELD

A local anesthetic patch is widely used for relief of pain caused by a medical procedure such as a puncture with an injection needle or an intravenous indwelling needle and a skin minor surgery. For example, a lidocaine tape is commercially available in Japan. The anesthetic effect of a lidocaine tape is, however, not sufficient.

In addition, an anesthetic cream containing a mixture of lidocaine and prilocaine as active pharmaceutical ingredients is also commercially available in Japan. A higher anesthetic effect of the cream than that of an anesthetic lidocaine tape is confirmed (Patent document 1). The cream, however, has problems of a complicated procedure that needs time and efforts. For example, the cream has to be spread so that the cream is thickly applied on the skin, an ODT: occlusive dressing technique using a film or the like is needed, and the cream has to be cleanly wiped away after use.

A gel that has a trade name of AMETOP^{(R)} and that contains tetracaine as an active ingredient is commercially available in Europe and the United States, and an anesthetic effect thereof is expressed more rapidly than that of an anesthetic cream. On the one hand, since the gel has to be wiped away after use similarly to a cream, there is a need to develop a product that can be handled more easily in the medical field.

Thus, a patch is considered to be used. For example, Patent document 2 discloses a transdermally administered drug patch that has less irritation to the skin, that is excellent in a medicinal property and that contains a drug, a transdermal penetration accelerator, an adhesive resin and an additive in an adhesive layer. Patent document 3 discloses a patch that has a sufficient drug solubility property, a transdermal penetration property and a sufficient adhesive property to the skin, that has less irritation to the skin, that contains a drug, a thermoplastic elastomer and a higher fatty acid ester in an adhesive layer, and in which a ratio of the higher fatty acid ester to the thermoplastic elastomer is adjusted and in which a content amount of a tackifier is reduced.

Patent document 4 describes a support made of nonwoven fabric or woven fabric, and a paste containing 100 parts by weight of a rubber adhesive, 70 to 150 parts by weight of a local anesthetic, and 20 to 80 parts by weight of a plasticizer formed on the support.

Patent document 5 describes an external preparation containing lidocaine lactate and diclofenac, wherein the content of lidocaine lactate is 2 to 5 times the molar amount of diclofenac.

Patent document 6 relates to a patch obtained by irradiating a composition containing a rubber elastomer, a tackifier, a softener and a medicinal component with an electron beam.

Patent document 7 relates to an adhesive sheet for attachment to the skin having an adhesive layer atop a support body, wherein the adhesive layer comprises a thermoplastic elastomer and a branched long-chain alcohol having a total number of carbons of 13 or more.

### PRIOR ART DOCUMENT

### PATENT DOCUMENT

Patent document 1: JP S54-101414 A
Patent document 2: JP H6-205839 A
Patent document 3: WO 2017/099246
Patent document 4: JP 2002-080350 A
Patent document 5: WO 2018/230687 A1
Patent document 6: JP 2008-266175 A
Patent document 7: WO 2019/142940 A1

### DISCLOSURE OF THE INVENTION

### PROBLEMS TO BE SOLVED BY THE INVENTION

A patch containing a drug in an adhesive layer has developed as described above. Nevertheless, though Patent documents 2 and 3 disclose an example of a local anesthetic agent as a drug to be contained in an adhesive layer, in fact, a patch described in Patent document 2 mainly contains a non-steroid anti-inflammatory drug such as ketoprofen, a patch described in Patent document 3 mainly contains scopolamine as an anticholinergic drug, donepezil hydrochloride as a progression inhibitor of Alzheimer dementia or the like, or a non-steroid anti-inflammatory drug, and Patent documents 2 and 3 do not disclose a specific patch containing a local anesthetic agent. In addition, a local anesthetic agent-containing patch excellent in all of a skin permeability, a cohesion force and an adhesive property has not been still developed.

Accordingly, the objective of the present invention is to provide a local anesthetic agent-containing patch having a skin permeability, cohesion force and adhesive property practical as a pharmaceutical product.

### MEANS FOR SOLVING THE PROBLEMS

The inventors of the present invention repeated intensive studies in order to solve the above-described problems. As a result, the inventors completed the present invention by finding that a local anesthetic agent-containing patch having an adhesive property and a cohesion force practical as a pharmaceutical product and a sufficient skin permeability can be obtained by adding at least a local anesthetic agent, a thermoplastic elastomer, a higher fatty acid ester, and a polyol fatty acid monoester in an adhesive layer and appropriately adjusting a ratio of the higher fatty acid ester to the thermoplastic elastomer.

The present invention is hereinafter described.

The present invention relates to the following aspects [1] to [14].
[1] A patch,
   comprising a backing layer, and an adhesive layer on the backing layer,
   wherein the adhesive layer comprises at least a local anesthetic agent, a thermoplastic elastomer, a higher fatty acid ester, and a polyol fatty acid monoester,
   a ratio of the higher fatty acid ester to 100 mass parts of the thermoplastic elastomer is 25 mass parts or more and 200 mass parts or less,
   a carbon number in an ester part of the higher fatty acid ester is 12 or more and 30 or less,
   the higher fatty acid is capric acid or a fatty acid having a carbon number of 12 or more and 30 or less, and
   the adhesive layer comprises 5 mass% or more of a tackifier.
[2] The patch according to the above [1], wherein a ratio of the higher fatty acid ester to 100 mass parts of the thermoplastic elastomer is 30 mass parts or more and 150 mass parts or less.
[3] The patch according to the above [1] or [2], wherein a content amount of the higher fatty acid ester in the adhesive layer is 60 mass% or less.
[4] The patch according to any one of the above [1] to [3], wherein the thermoplastic elastomer is a styrene block copolymer.
[5] The patch according to the above [4], wherein the styrene block copolymer is a mixture of a styrene-isoprene-styrene block copolymer and a styrene-isoprene block copolymer.
[6] The patch according to the above [5], wherein a ratio of the styrene-isoprene block copolymer in the mixture is 50 mass% or more.
[7] The patch according to any one of the above [4] to [6], wherein a viscosity of a 25 mass% toluene solution of the styrene block copolymer at 25°C is 500 mPa·s or more and 2000 mPa·s or less.
[8] The patch according to any one of the above [1] to [7], wherein the polyol fatty acid monoester is a propylene glycol fatty acid monoester.
[9] The patch according to any one of the above [1] to [8], wherein the adhesive layer further comprises a higher alcohol.
[10] The patch according to the above [9], wherein a carbon number of the higher alcohol is 12 or more and 30 or less.
[11] The patch according to the above [9] or [10], wherein the higher alcohol is oleyl alcohol and/or lauryl alcohol.
[12] The patch according to any one of the above [1] to [11], wherein the local anesthetic agent is one or more local anesthetic agents selected from the group consisting of tetracaine, lidocaine, prilocaine, bupivacaine, mepivacaine, benzocaine, levobupivacaine and ropivacaine.
[13] The patch according to any one of the above [1] to [11], wherein the local anesthetic agent is tetracaine.
   The following uses and methods are also described herein.
[14] Use of a local anesthetic agent, a thermoplastic elastomer, a higher fatty acid ester, and a polyol fatty acid monoester for local anesthesia,
   wherein an adhesive layer of a patch comprises the local anesthetic agent, the thermoplastic elastomer, the higher fatty acid ester, and the polyol fatty acid monoester,
   the patch comprises the adhesive layer and a backing layer, and the adhesive layer is placed on the backing layer, and
   a ratio of the higher fatty acid ester to 100 mass parts of the thermoplastic elastomer is 25 mass parts or more and 200 mass parts or less.
[15] The use according to the above [14], wherein a ratio of the higher fatty acid ester to 100 mass parts of the thermoplastic elastomer is 30 mass parts or more and 150 mass parts or less.
[16] The use according to the above [14] or [15], wherein a content amount of the higher fatty acid ester in the adhesive layer is 60 mass% or less.
[17] The use according to any one of the above [14] to [16], wherein a carbon number in an ester part of the higher fatty acid ester is 12 or more and 30 or less.
[18] The use according to any one of the above [14] to [17], wherein the thermoplastic elastomer is a styrene block copolymer.
[19] The use according to the above [18], wherein the styrene block copolymer is a mixture of a styrene-isoprene-styrene block copolymer and a styrene-isoprene block copolymer.
[20] The use according to the above [19], wherein a ratio of the styrene-isoprene block copolymer in the mixture is 50 mass% or more.
[21] The use according to any one of the above [18] to [20], wherein a viscosity of a 25 mass% toluene solution of the styrene block copolymer at 25°C is 500 mPa·s or more and 2000 mPa·s or less.
[22] The use according to any one of the above [14] to [21], wherein the adhesive layer comprises 5 mass% or more of a tackifier.
[23] The use according to any one of the above [14] to [22], wherein the polyol fatty acid monoester is a propylene glycol fatty acid monoester.
[24] The use according to any one of the above [14] to [23], wherein the adhesive layer further comprises a higher alcohol.
[25] The use according to the above [24], wherein a carbon number of the higher alcohol is 12 or more and 30 or less.
[26] The use according to the above [24] or [25], wherein the higher alcohol is oleyl alcohol and/or lauryl alcohol.
[27] The use according to any one of the above [14] to [26], wherein the local anesthetic agent is one or more local anesthetic agents selected from the group consisting of tetracaine, lidocaine, prilocaine, bupivacaine, mepivacaine, benzocaine, levobupivacaine and ropivacaine.
[28] The use according to any one of the above [14] to [26], wherein the local anesthetic agent is tetracaine.
[29] A local anesthetic method,
   comprising the step of applying a patch to a skin,
   wherein the patch comprises a backing layer, and an adhesive layer on the backing layer,
   the adhesive layer comprises at least a local anesthetic agent, a thermoplastic elastomer, a higher fatty acid ester, and a polyol fatty acid monoester, and
   a ratio of the higher fatty acid ester to 100 mass parts of the thermoplastic elastomer is 25 mass parts or more and 200 mass parts or less.
[30] The local anesthetic method according to the above [29], wherein a ratio of the higher fatty acid ester to 100 mass parts of the thermoplastic elastomer is 30 mass parts or more and 150 mass parts or less.
[31] The local anesthetic method according to the above [29] or [30], wherein a content amount of the higher fatty acid ester in the adhesive layer is 60 mass% or less.
[32] The local anesthetic method according to any one of the above [29] to [31], wherein a carbon number in an ester part of the higher fatty acid ester is 12 or more and 30 or less.
[33] The local anesthetic method according to any one of the above [29] to [32], wherein the thermoplastic elastomer is a styrene block copolymer.
[34] The local anesthetic method according to the above [33], wherein the styrene block copolymer is a mixture of a styrene-isoprene-styrene block copolymer and a styrene-isoprene block copolymer.
[35] The local anesthetic method according to the above [34], wherein a ratio of the styrene-isoprene block copolymer in the mixture is 50 mass% or more.
[36] The local anesthetic method according to any one of the above [33] to [35], wherein a viscosity of a 25 mass% toluene solution of the styrene block copolymer at 25°C is 500 mPa·s or more and 2000 mPa·s or less.
[37] The local anesthetic method according to any one of the above [29] to [36], wherein the adhesive layer comprises 5 mass% or more of a tackifier.
[38] The local anesthetic method according to any one of the above [29] to [37], wherein the polyol fatty acid monoester is a propylene glycol fatty acid monoester.
[39] The local anesthetic method according to any one of the above [29] to [38], wherein the adhesive layer further comprises a higher alcohol.
[40] The local anesthetic method according to the above [39], wherein a carbon number of the higher alcohol is 12 or more and 30 or less.
[41] The local anesthetic method according to the above [39] or [40], wherein the higher alcohol is oleyl alcohol and/or lauryl alcohol.
[42] The local anesthetic method according to any one of the above [29] to [41], wherein the local anesthetic agent is one or more local anesthetic agents selected from the group consisting of tetracaine, lidocaine, prilocaine, bupivacaine, mepivacaine, benzocaine, levobupivacaine and ropivacaine.
[43] The local anesthetic method according to any one of the above [29] to [41], wherein the local anesthetic agent is tetracaine.
[44] A local anesthetic method, comprising the step of applying the patch according to any one of the above [1] to [13] to a skin.

### EFFECT OF THE INVENTION

The present invention can provide a local anesthetic agent-containing patch having a practical adhesive property and a practical cohesion force as a pharmaceutical product and a sufficient skin permeability.

### MODE FOR CARRYING OUT THE INVENTION

An adhesive layer is formed on a backing layer in the patch of the present invention. The "backing layer" is not particularly restricted in the present invention, and a backing layer that is widely used for an adhesive sheet for skin application and a percutaneous absorption product may be used. An example of the backing layer includes a stretchable or a non-stretchable woven fabric or non-woven fabric composed of polyethylene, polypropylene, polyethylene terephthalate or the like; a film composed of a polyester such as polyethylene terephthalate, a polyolefin such as polyethylene and polypropylene, polyurethane, ethylene-vinyl acetate copolymer, polyvinyl chloride or the like; a foamed backing layer composed of polyolefin, polyurethane or the like. One of the backing layer may be used alone, or a laminate prepared by laminating a plurality of backing layers may be used. The above-described woven fabric, non-woven fabric, film or the like that constitute the backing layer may contain an antistatic agent to prevent static electricity from accumulating on the backing layer. A non-woven fabric, a woven fabric, or a laminate of a non-woven fabric or a woven fabric and a film may be used as the backing layer for a good anchoring property with an adhesive layer. When a laminate of a film and a non-woven fabric or a woven fabric is used as the backing layer, the non-woven fabric or woven fabric is preferably placed on the side to be contacted with an adhesive layer.

A thickness of the film as the backing layer is generally 10 µm or more and 100 µm or less and preferably 15 µm or more and 50 µm or less, and a thickness of the woven fabric, non-woven fabric and foamed backing layer is generally 50 µm or more and 2,000 µm or less and preferably 100 µm or more and 1,000 µm or less.

The patch of the present invention may comprise a release liner that is general in this technical field. In other words, the backing layer, an adhesive layer and a release liner may be laminated in this order in the patch of the present invention. A glassine paper; a polymer film composed of a polyolefin such as polyethylene and polypropylene, a polyester such as polyethylene terephthalate, and polystyrene; an aluminum film; a foamed polyethylene film and a foamed polypropylene film; and a laminate of two or more of the above examples can be used as the release liner. The release liner subjected to silicone processing, fluororesin processing, embossing, hydrophilicizing processing and hydrophobicizing may be also used. A thickness of the release liner is generally 10 µm or more and 200 µm or less and preferably 15 µm or more and 150 µm or less.

An adhesive layer of the patch according to the present invention comprises at least (a) a local anesthetic agent as a drug, (b) a thermoplastic elastomer, (c) a higher fatty acid ester and (d) a polyol fatty acid monoester.

### (a) Local anesthetic agent

The "local anesthetic agent" usable in the present invention means a drug to obtund or erase the target local perception and to relieve a local pain, and is not particularly restricted as long as the local anesthetic agent has a basic skeleton containing an aromatic ring, an alkyl chain and an amino group and has a structure formed by connecting the aromatic ring and the alkyl chain through an ester bond or an amide bond. An example of the local anesthetic agent includes one or more local anesthetic agents selected from the group consisting of tetracaine, lidocaine, prilocaine, bupivacaine, mepivacaine, benzocaine, levobupivacaine and ropivacaine, and the local anesthetic agent is preferably one or more local anesthetic agents selected from the group consisting of tetracaine, lidocaine and prilocaine and more preferably tetracaine.

The local anesthetic agent usable as a raw material is not particularly restricted and may be a free form or a pharmaceutically acceptable salt. Such a pharmaceutically acceptable salt is not particularly restricted and may be an inorganic salt or an organic salt. Only one kind of the salt may be used, or two or more kinds of the salts may be used in combination. In addition, a free form and a salt may be mixed to be used. An example of the inorganic salt includes hydrochloride, hydrobromide, nitrate, sulfate and phosphate. An example of the organic salt includes formate, acetate, trifluoroacetate, propionate, lactate, tartrate, oxalate, fumarate, maleate, citrate, malonate and methanesulfonate. A free form or a hydrochloride is preferred in terms of availability, and a free form is more preferred in terms dispersibility in an adhesive.

A content amount of the local anesthetic agent in the adhesive layer of the patch according to the present invention, i.e. a ratio of the local anesthetic to 100 mass% of the total constituent components of the adhesive layer, is preferably 0.5 mass% or more and 30 mass% or less in terms of ensuring a dispersibility in the adhesive layer and a good skin permeability. The content ratio is more preferably 1 mass% or more, even more preferably 3 mass% or more, and more preferably 20 mass% or less, even more preferably 15 mass% or less.

### (b) Thermoplastic elastomer

The "thermoplastic elastomer" used in the present invention means a thermoplastic elastomer that becomes soft and fluid by heat and that returns to a rubbery elastomer by cooling, and is exemplified by various thermoplastic elastomers such as urethane elastomer, acrylate elastomer, styrene elastomer and olefin elastomer. In particular, a styrene thermoplastic elastomer, particularly a styrene block copolymer, is preferably used from the viewpoint of achieving both of sufficient skin adhesiveness and low dermal irritation.

An example of the styrene block copolymer as the thermoplastic elastomer specifically includes styrene-butadiene block copolymer, styrene-butadiene-styrene block copolymer, styrene-isoprene block copolymer, styrene-isoprene-styrene block copolymer, styrene-ethylene/butylene block copolymer, styrene-ethylene/butylene-styrene block copolymer, styrene-ethylene/propylene block copolymer, styrene-ethylene/propylene-styrene block copolymer, styrene-isobutylene block copolymer and styrene- isobutylene-styrene block copolymer. The above-described "ethylene/butylene" represents a copolymer block of ethylene and butylene, and the "ethylene/propylene" represents a copolymer block of ethylene and propylene. One of the styrene block copolymers may be used alone, or two or more may be used in combination.

One or more selected from the group consisting of a styrene-isoprene-styrene block copolymer and a styrene-isoprene block copolymer are preferably used and a mixture of a styrene-isoprene block copolymer and a styrene-isoprene-styrene block copolymer is particularly preferably used among the above-described styrene block copolymer from the viewpoint of the compatibility between a sufficient adhesiveness to the akin and a suppression of a remained adhesive due to an improved cohesive force in the adhesive layer and additionally availability and handleability. A ratio of the styrene-isoprene block copolymer in the mixture is preferably 50 mass% or more, more preferably 60 mass% or more, and preferably 90 mass% or less, more preferably 80 mass% or less.

A content amount of styrene in the styrene-isoprene-styrene block copolymer is preferably 5 mass% or more and 60 mass% or less and more preferably 10 mass% or more and 50 mass% or less for the objective of the present invention. In addition, a weight average molecular weight of the styrene-isoprene-styrene block copolymer measured by a gel permeation chromatography (GPC) is preferably 20,000 or more and 500,000 or less and more preferably 30,000 or more and 300,000 or less. A content amount of styrene in the styrene-isoprene block copolymer is preferably 5 mass% or more and 50 mass% or less and more preferably 10 mass% or more and 40 mass% or less. In addition, a weight average molecular weight of the styrene-isoprene block copolymer measured by a gel permeation chromatography (GPC) is preferably 10,000 or more and 500,000 or less and more preferably 20,000 or more and 300,000 or less.

A solution viscosity of 25 mass% toluene solution of the styrene block copolymer at 25°C is preferably 500 mPa·s or more and 2000 mPa·s or less from the viewpoint of good balance of an adhesive substance. The viscosity is more preferably 500 mPa·s or more, even more preferably 900 mPa·s or more, and more preferably 1800 mPa· s or less. The above described "solution viscosity of 25 mass% toluene solution at 25°C" means a value measured on the basis of the viscosity measuring method for styrene-isoprene-styrene block copolymer described in page 395 of "Japanese Pharmaceutical Excipients 2013" published by Yakuji Nippo.

The styrene-isoprene-styrene block copolymer and the styrene-isoprene block copolymer respectively produced by a publically known method may be used. In addition, commercially available products of the styrene-isoprene-styrene block copolymer and the styrene-isoprene block copolymer that satisfy the above-described characteristics may be used. A mixture of the styrene-isoprene-styrene block copolymer and the styrene-isoprene block copolymer is also commercially available, and a commercially available product of a mixture containing the styrene-isoprene-styrene block copolymer and the styrene-isoprene block copolymer in the above-described mixing ratio that satisfies the above-described characteristics may be preferably used.

An example of the commercially available product of the styrene block copolymer includes "KRATON^{(R)} D1111", "KRATON^{(R)} D1163", "KRATON^{(R)} D1113" and "KRATON^{(R)} D1119" manufactured by KRATON POLYMERS; "JSR^{(R)} SIS5229", "JSR^{(R)} SIS5002", "JSR^{(R)} SIS5403" and "JSR^{(R)} SIS5505" manufactured by JSR; and "Quintac^{(R)} 3421", "Quintac^{(R)} 3433N", "Quintac^{(R)} 3520", "Quintac^{(R)} 3450" and "Quintac^{(R)} 3270" manufactured by Zeon. Among the above examples, "KRATON^{(R)} D1163", "KRATON^{(R)} D1113", "JSR^{(R)} SIS5403", "JSR^{(R)} SIS5505", "Quintac^{(R)} 3433N" and "Quintac^{(R)} 3520" are preferred and "JSR^{(R)} SIS5505" and/or "Quintac^{(R)} 3520" is particularly preferred from the viewpoint of the mixing ratio and the solution viscosity of the styrene-isoprene-styrene block copolymer and the styrene-isoprene block copolymer.

A content amount of the thermoplastic elastomer in the adhesive layer, in other words, a ratio of the thermoplastic elastomer to 100 mass% of the total constituent components of the adhesive layer, is preferably 20 mass% or more and 70 mass% or less. When the ratio is 20 mass% or more, a form of the adhesive layer may be maintained more surely. When the ratio is 70 mass% or less, an adhesive force of the adhesive layer to the skin may be exerted more surely. The content amount is more preferably 25 mass% or more, even more preferably 30 mass% or more, and more preferably 65 mass% or less, even more preferably 60 mass% or less.

### (c) Higher fatty acid ester

The "higher fatty acid ester" in the present invention means a compound formed by an ester bond between a carboxy group of a higher fatty acid and an aliphatic alcohol. The higher fatty acid ester has an effect to appropriately plasticize the thermoplastic elastomer and contributes to an adhesive force. In addition, the higher fatty acid ester contributes to an improvement of a solubility and a suppression of crystal deposition of the local anesthetic agent, since the higher fatty acid ester has an appropriate affinity for the local anesthetic agent.

The higher fatty acid that constitutes the higher fatty acid ester may be linear or branched. The higher fatty acid may be saturated or unsaturated, and a saturated higher fatty acid is preferred from the view point of a plasticizing effect and a thermal stability of the thermoplastic elastomer. The higher fatty acid is capric acid or a fatty acid having a carbon number of the higher fatty acid of 12 or more and 30 or less, more preferably 14 or more, even more preferably 16 or more, and preferably 24 or less, even more preferably 20 or less.

An example of the saturated higher fatty acid includes capric acid (carbon number: 10), lauric acid (carbon number: 12), myristic acid (carbon number: 14), palmitic acid (carbon number: 16), stearic acid (carbon number: 18), isostearic acid (carbon number: 18), arachidic acid (carbon number: 20), behenic acid (carbon number: 22), lignoceric acid (carbon number: 24), cerotic acid (carbon number: 26), montanic acid (carbon number: 28) and melissic acid (carbon number: 30). Myristic acid, palmitic acid and stearic acid are preferred among the above examples.

An example of the unsaturated higher fatty acid includes palmitoleic acid (carbon number: 16), oleic acid (carbon number: 18), linoleic acid (carbon number: 18), (9,12,15)-linolenic acid (carbon number: 18), (6,9,12)-linolenic acid (carbon number: 18) and eleostearic acid (carbon number: 18). Oleic acid and linoleic acid are preferred among the above examples.

An aliphatic alcohol that constitutes the higher fatty acid ester is preferably a saturated or unsaturated aliphatic alcohol having a carbon number of 12 or more and 30 or less, and is exemplified by cetanol, myristyl alcohol, hexyldecanol, oleyl alcohol and octyldodecanol. A carbon number of the aliphatic alcohol corresponds to a carbon number of the ester part of the higher fatty acid ester. A carbon number of the aliphatic alcohol is 12 or more and 30 or less. When the carbon number is 12 or more, a plasticizing effect by the aliphatic alcohol may be exerted more surely. On the one hand, when the carbon number is 30 or less, a solubility of the local anesthetic agent may be sufficiently ensured.

A preferred example of the higher fatty acid ester specifically includes a myristate ester such as octyldodecyl myristate; a palmitate ester; a stearate ester; an oleate ester such as octyldodecyl oleate and oleyl oleate; and a linoleate ester.

A ratio of the higher fatty acid ester to 100 mass parts of the thermoplastic elastomer in the adhesive layer is 25 mass parts or more and 200 mass parts or less. When the ratio is 25 mass parts or more, a good adhesive force of the adhesive layer and a solubility of the local anesthetic agent may be exerted more surely. When the ratio is 200 mass parts or less, a form of the adhesive layer may be maintained more surely. The ratio is more preferably 30 mass parts or more and 150 mass parts or less. A ratio of the higher fatty acid ester in the adhesive layer of the patch according to the present invention is preferably 10 mass% or more, more preferably 15 mass% or more, even more preferably 20 mass% or more, and preferably 70 mass% or less, more preferably 65 mass% or less, even more preferably 60 mass% or less for a similar reason.

### (d) Polyol fatty acid monoester

The " polyol fatty acid monoester " in the present invention means a compound formed by an ester bond between one hydroxy group of the polyol and the fatty acid. The polyol fatty acid monoester contributes to an improvement of a drug solubility without extremely decreasing a cohesive force of an adhesive base agent and has an effect to accelerate a transdermal absorption of the local anesthetic agent, particularly tetracaine.

An example of the polyol that constitutes the polyol fatty acid monoester includes a diol such as ethylene glycol, propylene glycol and butylene glycol; a trivalent alcohol such as glycerin; and a tetravalent alcohol such as pentaerythritol. The fatty acid that constitutes a propylene glycol fatty acid monoester is preferably a fatty acid having a carbon number of 8 or more and 18 or less, and is exemplified by capric acid, caprylic acid, myristic acid, palmitic acid, stearic acid, isostearic acid, oleic acid and linoleic acid. A preferred specific example of the polyol fatty acid monoester includes propylene glycol monocaprylate and propylene glycol monolaurate.

A content amount of the polyol fatty acid monoester in the adhesive layer, in other words, a ratio of the polyol fatty acid monoester to 100 mass% of the total constituent components of the adhesive layer, is preferably 2 mass% or more and 30 mass% or less. When the ratio is 2 mass% or more, a solubility and an absorption enhancement effect of the local anesthetic agent may be improved more surely. When the ratio is 30 mass% or less, a cohesive force and an adhesive force of the adhesive layer may be ensured more surely. The ratio is more preferably 5 mass% or more.

A component other than the local anesthetic agent, thermoplastic elastomer, higher fatty acid ester and polyol fatty acid monoester may be added to the adhesive layer of the patch according to the present invention. Such an optional component is not particularly restricted as long as the optional component is a general component to be added in an adhesive layer of a patch, and is exemplified by higher alcohol, solvent, carboxylate salt, lactone, surfactant, filler and crystallization inhibitor. Further, in the patch of the present invention, the adhesive layer comprises 5 mass% or more of a tackifier.

### (e) Tackifier

The "tackifier" in the present invention means a component to enhance an adhesibility of the adhesive layer, and is not restricted as long as the tackifier is widely used for a general patch, and is exemplified by rosin resin, polyterpene resin, coumarone-indene resin, petroleum resin, terpene resin, terpene-phenol resin and aliphatic cyclic saturated hydrocarbon resin.

A content amount of the tackifier in the adhesive layer, in other words, a ratio of the tackifier to 100 mass% of the total constituent components of the adhesive layer, may be appropriately adjusted, and it is particularly adjusted to 5 mass% or more and preferably 50 mass% or less. When the ratio is 5 mass% or more, the adhesibility needed to obtain a sufficient drug efficacy can be ensured more surely. When the ratio is 50 mass% or less, a decrease of a drug releasing amount and an increase of skin irritation can be inhibited more surely. The ratio is more preferably 7 mass% or more and even more preferably 10 mass% or more.

### (f) Higher alcohol

The "higher alcohol" in the present invention means a component to improve a solubility of the local anesthetic agent in the adhesive layer and a transdermal absorption property of the local anesthetic agent, and is exemplified by a higher saturated aliphatic alcohol that has a carbon number of about 12 or more and about 30 or less and that is liquid under ordinary temperature, such as lauryl alcohol and isostearyl alcohol; and a higher unsaturated aliphatic alcohol that has a carbon number of about 12 or more and about 30 or less and that is liquid under ordinary temperature, such as oleyl alcohol. The higher alcohol is preferably oleyl alcohol and/or lauryl alcohol from the viewpoint to enhance the effect to improve a solubility of the local anesthetic agent and to improve an effect to enhance a transdermal absorption.

A content amount of the higher alcohol in the adhesive layer, in other words, a ratio of the higher alcohol to 100 mass% of the total constituent components of the adhesive layer, may be appropriately adjusted, and may be adjusted to, for example, 1 mass% or more and 30 mass% or less. When the ratio is 1 mass% or more, a solubility of the local anesthetic agent in the adhesive layer and a transdermal absorption property of the local anesthetic agent may be improved more surely. When the ratio is 30 mass% or less, a cohesive force and an adhesibility of the adhesive layer may be ensured more surely. The ratio is preferably 3 mass% or more.

### (g) Solvent

The "solvent" in the present invention means a component that is a liquid under an atmospheric temperature and an atmospheric pressure and that shows an appropriate solubility to an adhesive component. The solvent usable in the present invention is not particularly restricted, and is exemplified by one or more solvents selected from the group consisting of an ester solvent, an alcohol solvent, an amide solvent and a liquid organic acid.

A use amount of the solvent may be appropriately adjusted, and for example, a content amount of the solvent in the adhesive layer, in other words, a ratio of the solvent to 100 mass% of the total constituent components of the adhesive layer, may be adjusted to 0.1 mass% or more and 20 mass% or less and preferably 0.5 mass% or more and 15 mass% or less.

### (g1) Ester solvent

An ester solvent is preferred among the solvent from the viewpoint to improve a solubility of the local anesthetic agent in the adhesive layer and a transdermal absorption property of the local anesthetic agent. An example of the ester solvent includes a diester of a diol and a carboxylic acid, a triglyceride of a medium chain fatty acid, an ester of a multivalent carboxylic acid and a monovalent aliphatic alcohol, and a carbonate ester.

An example of the diester of diol and carboxylic acid includes a diester prepared from propylene glycol and caprylic acid, capric acid, lauric acid or oleic acid.

The triglyceride of medium chain fatty acid means a triglyceride composed of an aliphatic acid having a carbon number of 6 or more and 12 or less and glycerin. An example of the aliphatic acid includes caproic acid, caprylic acid, capric acid and lauric acid. A caprylic acid triglyceride, a triglyceride mixture of caprylic acid and capric acid, and a triglyceride mixture of caprylic acid, capric acid and lauric acid that are liquids under an atmospheric temperature may be used in the present invention. In addition, a fat and oil that is liquid under an atmospheric temperature and that contains much of the above triglycerides may be also used. An example of the fat and oil includes peanut oil, olive oil and castor oil.

A commercially available pharmaceutical product may be used as the triglyceride of medium chain fatty acid that is liquid under an atmospheric temperature or a fat and oil containing the triglyceride of medium chain fatty acid that is liquid under an atmospheric temperature in the present invention.

An example of the ester of multivalent carboxylic acid and monovalent aliphatic alcohol includes a diester that is composed of a dicarboxylic acid having a carbon number of 2 or more and 12 or less and a monovalent aliphatic alcohol having a carbon number of 1 or more and 20 or less and that is liquid under an atmospheric temperature. An example of the diester includes an adipate diester that is liquid under an atmospheric temperature, such as diethyl adipate and diisopropyl adipate; and a sebacate diester that is liquid under an atmospheric temperature, such as diethyl sebacate, diisopropyl sebacate and dioctyldodecy sebacate.

The carbonate ester is exemplified by a cyclic carbonate ester of carbonic acid and a diol having a carbon number of 2 or more and 10 or less, such as ethylene carbonate, propylene carbonate and vinylene carbonate, and is preferably propylene carbonate.

The triglyceride of medium chain fatty acid mixture, sebacate diester and carbonate ester are preferred and the triglyceride mixture of caprylic acid and capric acid, diethyl sebacate and propylene carbonate are more preferred among the above-described ester solvents.

### (g2) Alcohol solvent

An example of the alcohol solvent includes a multivalent alcohol that is liquid under an atmospheric temperature, such as ethylene glycol, propylene glycol, glycerin, 1,3-butanediol and polyethylene glycol having a molecular weight of 100 or more and 600 or less; a monoalkyl ether of a multivalent alcohol, such as diethylene glycol monoethyl ether; and a monofatty acid ester of a multivalent alcohol, such as glycerol monolinoleate and glycerol monooleate.

In particular, one or more alcohol solvents selected from the group consisting of ethylene glycol, propylene glycol, glycerin, 1,3-butanediol and diethylene glycol monoethyl ether are preferred from the viewpoint to improve a solubility of the local anesthetic agent.

### (g3) Amide solvent

An example of the amide solvent includes a pyrrolidone such as N-methyl-2-pyrrolidone and 2-pyrrolidone; an imidazolidinone such as 1,3-dimethyl-2-imidazolidinone; an N-substituted toluidine such as crotamiton; and an alkane amide such as formamide, N-methylformamide, N,N-dimethylformamide, N-methylacetamide, N,N-dimethylacetamide and N-methylpropanamide.

The above-described amide solvent is preferably N-methyl-2-pyrrolidone, crotamiton, N,N-dimethylformamide and N,N-dimethylacetamide and more preferably N-methyl-2-pyrrolidone and/or crotamiton from the viewpoint to improve a solubility, a dispersibility and a transdermal absorption property of the local anesthetic agent.

### (g4) Liquid organic acid

An example of the liquid organic acid includes an aliphatic monocarboxylic acid such as acetic acid, propionic acid, butyric acid, valeric acid, isovaleric acid, caproic acid, enanthic acid (heptanoic acid), caprylic acid and pelargonic acid (nonanoic acid); an aliphatic unsaturated monocarboxylic acid such as oleic acid, linoleic acid, arachidonic acid and docosahexaenoic acid; a hydroxycarboxylic acid such as lactic acid; a liquid carboxylic acid substituted by an alkoxy group, such as methoxyacetic acid; and a sulfonic acid such as methanesulfonic acid. Lactic acid may be L-lactic acid, D-lactic acid, lactic anhydride or a mixture thereof.

The liquid organic acid has a function to assist a dissolution of the basic local anesthetic agent and has an effect to enable the local anesthetic agent to be contained in the adhesive layer in a high concentration and to improve a dispersibility and further a transdermal absorption property. Lactic acid of Japanese Pharmacopoeia grade, oleic acid and isostearic acid are preferably used and lactic acid of Japanese Pharmacopoeia grade is more preferably used from the above-described viewpoint.

One or two or more of the liquid organic acids may be selected from the above-described liquid organic acids as needed to be contained in the adhesive layer in the present invention. A content amount of the liquid organic acid in the adhesive layer, in other words, a ratio of the liquid organic acid to 100 mass% of the total constituent components of the adhesive layer, is preferably 0.1 mass% or more and 20 mass% or less and more preferably 0.5 mass% or more and 15 mass% or less.

### (h) Carboxylate salt

An example of the carboxylate salt mixed in the adhesive layer of the patch according to the present invention includes salts of an aliphatic monocarboxylic acid, an aliphatic cyclic monocarboxylic acid and an aliphatic dicarboxylic acid.

An example of the aliphatic monocarboxylic acid includes a short chain carboxylic acid having a carbon number of 2 or more and 7 or less, such as acetic acid, butyric acid and hexanoic acid; a medium chain fatty acid having a carbon number of 8 or more and 11 or less, such as octanoic acid and decanoic acid; a long chain fatty acid having a carbon number of 12 or more, such as myristic acid, stearic acid, isostearic acid and oleic acid; a hydroxy monocarboxylic acid such as glycolic acid, lactic acid, 3-hydroxybutyric acid and mandelic acid; a monocarboxylic acid substituted by an alkoxy group, such as methoxyacetic acid; and a keto monocarboxylic acid such as levulinic acid.

An example of the aliphatic cyclic monocarboxylic acid includes an aliphatic cyclic monocarboxylic acid having a carbon number of 6 or more and 8 or less, such as cyclohexanecarboxylic acid.

An example of the aliphatic dicarboxylic acid includes sebacic acid, adipic acid, malic acid, maleic acid and fumaric acid.

An example of the preferred carboxylic acid includes a long chain carboxylic acid having a carbon number of 12 or more and a hydroxy monocarboxylic acid, such as myristic acid, stearic acid, isostearic acid, oleic acid and lactic acid. The carboxylic acid is more preferably oleic acid and/or lactic acid.

An example of a salt of the carboxylic acid includes an alkali metal salt such as sodium salt and potassium salt; an alkaline earth metal salt such as calcium salt; and an amine salt, and a sodium salt is preferably used from the viewpoint of easy availability, stability and an effect to improve a transdermal absorption property of the local anesthetic agent.

### (i) Lactone

An example of the lactone includes a 5-membered lactone such as ascorbic acid and isoascorbic acid.

The carboxylate salt and/or the lactone is preferably added to the adhesive layer of the patch according to the present invention from the viewpoint of an effect to improve a stability and a transdermal absorption property of the local anesthetic agent, and one or more selected from the group consisting of sodium oleate, sodium lactate, ascorbic acid and isoascorbic acid are preferably added.

When the patch of the present invention contains the carboxylate salt and/or the lactone, a content amount thereof in the adhesive layer is not particularly restricted. For example, the content amount to 1 mole of the local anesthetic agent is preferably 0.1 mole or more and 5 mole or less and more preferably 0.2 mole or more and 3 mole or less. When the ratio of the carboxylate salt and/or the lactone to 1 mole of the local anesthetic agent is 0.1 mole or more, a sufficient effect to improve a transdermal absorption property of the local anesthetic agent may be obtained more surely. When the ratio is 5 mole or less, a preparation physical property such as adhesibility may be ensured more surely.

### (j) Surfactant

Any one of a non-ionic surfactant, an anionic surfactant, a cationic surfactant and an ampholytic surfactant can be used as a surfactant. An example of a non-ionic surfactant includes a polyoxyethylene fatty acid ester such as polyoxyethylene monolaurate; a polyoxyethylene sorbit fatty acid ester such as polyoxyethylene sorbit tetraoleate; a polyoxyethylene sorbitan fatty acid ester such as polyoxyethylene sorbitan monooleate, polyoxyethylene sorbitan monolaurate and polyoxyethylene sorbitan monopalmitate; a sorbitan fatty acid ester such as sorbitan monolaurate, sorbitan monooleate, sorbitan sesquioleate and sorbitan trioleate; a glycerin fatty acid ester such as glycerin monooleate, polyoxyethylene castor oil derivative and polyoxyethylene hydrogenated castor oil; a polyoxyethylene higher aliphatic alcohol ether such as polyoxyethylene lauryl ether and polyoxyethylene oleyl ether; a polyoxyethylene alkylphenyl ether such as polyoxyethylene nonyl phenyl ether; a polyoxyethylene alkylamino ether such as polyoxyethylene laurylamine and polyoxyethylene oleylamine; and a polyoxyethylene polyoxypropylene copolymer such as Pluronic^{(R)} L-31 and Pluronic^{(R)} L-44. An example of an anionic surfactant includes a sodium alkylsulfate such as sodium laurylsulfate. An example of a cationic surfactant includes an alkyltrimethylammonium salt and an alkyldimethylammonium salt. An example of an ampholytic surfactant includes an alkyldimethylamine oxide and an alkyl carboxy betain. One kind or two or more kinds of the surfactant may be selected to be used.

A non-ionic surfactant that is liquid under an atmospheric temperature is preferred, a sorbitan fatty acid ester that is liquid under an atmospheric temperature is more preferred, and sorbitan monolaurate is even more preferred among a surfactant from the viewpoint to improve a transdermal absorption property of the local anesthetic agent.

A content amount of the surfactant in the adhesive layer in the case where the surfactant is used in the present invention, in other words, a ratio of the surfactant to 100 mass% of the total constituent components of the adhesive layer, is preferably 0.01 mass% or more and 10 mass% or less and more preferably 0.1 mass% or more and 5 mass% or less.

### (k) Antioxidant agent

An example of an antioxidant agent includes dibutylhydroxytoluene, 4-dioxyphenol, tocopherol, a tocopherol ester derivative, ethylenediaminetetraacetic acid disodium salt, rutin, N,N-dimethylthiourea, L-cysteine, 1-thioglycerol and 2-mercaptobenzimidazole. In particular, dibutylhydroxytoluene is preferred. One antioxidant agent may be used alone or two or more antioxidant agents may be used in combination.

A content amount of the antioxidant agent is not particularly restricted, and the antioxidant agent may be contained in the range that a high transdermal permeability of the local anesthetic agent and a sufficient cohesive force and an adhesibility for the patch can be maintained. For example, a content amount of the antioxidant agent in the adhesive layer, in other words, a ratio of the antioxidant agent to 100 mass% of the total constituent components of the adhesive layer, may be adjusted to 10 mass% or less, and is preferably 5 mass% or less and more preferably 2 mass% or less. The antioxidant agent is an optional component, and a content amount of the antioxidant agent in the adhesive layer may be 0 mass%. The lower limit of the content amount is preferably 0.01 mass%.

### (l) Filler

A filler may be added to control a flexibility of the adhesive layer. An example of the filler includes a silicon compound such as anhydrous silicic acid, light anhydrous silicic acid and hydrous silicic acid; a cellulose derivative such as ethyl cellulose, methyl cellulose, hydroxypropyl cellulose and hydroxypropylmethylcellulose; a water-soluble polymer such as polyvinyl alcohol; an aluminum compound such as dried aluminum hydroxide gel and hydrous aluminum silicate; kaolin; and titanium oxide. One filler may be used alone or two or more fillers may be used in combination.

A content amount of the filler is not particularly restricted, and the filler may be added in the range that a high transdermal permeability of the local anesthetic agent and a sufficient cohesive force and an adhesibility for the patch can be maintained. For example, a content amount of the filler in the adhesive layer, in other words, a ratio of the filler to 100 mass% of the total constituent components of the adhesive layer, may be adjusted to 10 mass% or less, and is preferably 5 mass% or less and more preferably 2 mass% or less. The filler is an optional component, and a content amount of the filler in the adhesive layer may be 0 mass%. The lower limit of the content amount is preferably 0.01 mass%.

### (m) Crystallization inhibitor

A crystallization inhibitor may be added to prevent a crystal of the local anesthetic agent from being precipitated in the adhesive layer. An example of the crystallization inhibitor includes polyvinylpyrrolidone, vinyl acetate-vinylpyrrolidone copolymer and polyvinylcaprolactam-polyvinyl acetate-polyethylene glycol graft copolymer. One crystallization inhibitor may be used alone, or two or more crystallization inhibitors may be used in combination.

A content amount of the crystallization inhibitor is not particularly restricted, and the crystallization inhibitor may be added in the range that an adhesibility for the patch can be maintained. For example, a content amount of the crystallization inhibitor in the adhesive layer, in other words, a ratio of the crystallization inhibitor to 100 mass% of the total constituent components of the adhesive layer, may be adjusted to 0.01 mass% or more and 10 mass% or less, and is preferably 0.1 mass% or more and 5 mass% or less.

The patch of the present invention can be produced by an ordinary method. For example, the patch can be produced by mixing the constituent components of the adhesive layer or dissolving or dispersing the adhesive layer components in a low boiling point solvent other than the solvent constituting the adhesive layer to prepare a coating liquid for forming the adhesive layer, applying the coating liquid on the backing layer, and then drying the applied coating liquid. When a release liner is used, the release liner is pressed on the adhesive layer to be laminated. Alternatively, the above-described coating liquid is applied on a release liner and dried to form the adhesive layer on the release liner, and the backing layer is laminated and pressed on the adhesive layer to be adhered.

The low boiling point solvent for the coating liquid is preferably a solvent that can homogeneously dissolve or disperse the adhesive layer components, and is exemplified by an aromatic hydrocarbon such as toluene; an aliphatic cyclic hydrocarbon such as cyclohexane and methylcyclohexane; an aliphatic hydrocarbon such as hexane and heptane; an ether solvent such as tetrahydrofuran, diethyl ether and t-butyl methyl ether; a ketone solvent such as acetone, methyl ethyl ketone and methyl isobutyl ketone; an alcohol solvent such as ethanol, propanol and butanol; and an acetate ester solvent such as ethyl acetate, propyl acetate, isopropyl acetate, butyl acetate and isobutyl acetate. One of the solvent may be used alone, or two or more of the solvents may be used in combination. An aromatic hydrocarbon, an aliphatic cyclic hydrocarbon and an aliphatic hydrocarbon are preferably used alone or in combination, or an aromatic hydrocarbon, an aliphatic hydrocarbon and an acetate ester solvent are preferably used in an appropriate combination, since each of the constituent components of the adhesive layer can be successfully dissolved.

A coating liquid for forming the adhesive layer may be applied using a general coater such as roll coater, die coater, gravure roll coater, reverse roll coater, kiss-roll coater, dip roll coater, bar coater, knife coater and spray coater. The above coating liquid is preferably dried by heating, for example, at about 40°C or higher and 150 ° C or lower. A drying temperature, a drying time and a drying method may be adjusted depending on a used solvent and use amount thereof. A weight per a unit area of the adhesive layer after drying may be adjusted depending on a needed adhesiveness to the akin and a transdermal absorbability, and the range thereof by which adhesiveness to the akin can be exerted and by which the adhesive layer can be produced is preferably 10 g/m² or more and 1,000 g/m² or less, more preferably 20 g/m² or more and 800 g/m² or less, and even more preferably 30 g/m² or more and 600 g/m² or less.

The patch of the present invention may be used similarly to a general patch. For example, when the patch of the present invention contains a release liner, the release liner is peeled to expose the adhesive layer and the adhesive layer is attached on the skin. The number of uses of the patch according to the present invention may be appropriately adjusted depending on symptom, age, sex or the like of the subject, and 1 sheet or more and 10 sheets or less may be used per one time. An adhesion time per 1 sheet may be also appropriately adjusted and may be adjusted to, for example, 10 minutes or more and 1 hour or less.

The patch of the present invention is applied on the skin of an animal. The subject animal is exemplified by a human; livestock such as cow, pig, sheep and goat; and a pet animal such as dog and cat, and is preferably a human. When the patch is applied on an animal having much hair, the patch may be applied on a shaved part.

The present application claims the benefit of the priority date of Japanese patent application No. 2019-47009 filed on March 14, 2019. All of the contents of the Japanese patent application No. 2019-47009 filed on March 14, 2019.

### EXAMPLES

The present invention is hereinafter described in more detail with Examples and Comparative examples and is not restricted thereto.

### Examples 1 to 5, Reference Examples 6 and 7, Comparative examples 1 to 6: Production of patch

An amount of each component that constituted an adhesive layer was weighted in accordance with the composition described in Table 1. First, a styrene block copolymer was dissolved in toluene, and then octyldodecyl myristate, propylene glycol monocaprylate, a terpene resin and tetracaine were added thereto. The mixture was mixed and stirred to prepare a coating liquid for forming an adhesive layer.

The above coating liquid was applied on a polyethylene terephthalate (PET) film treated by silicone as a release liner and dried in an oven at 50°C for 60 minutes so that the thickness of the adhesive layer after drying became about 400 µm. Then, a PET film as a backing layer was laminated on the surface of the adhesive layer, and the laminate was cut into a size of 15 cm × 30 cm to obtain a patch.

**Table 1**

| | | Ex. 1 | Ex. 2 | Ex. 3 | Ex. 4 | Ex. 5 | Ex. 6 | Ex. 7 |
|---|---|---|---|---|---|---|---|---|
| Tetracaine | | 4.0 | 4.0 | 4.0 | 4.0 | 4.0 | 4.0 | 4.0 |
| Styrene block copolymer | "Quintac 3520" Zeon Corporation | 67.2 | 64.8 | 27.0 | 60.8 | 25.3 | 68.8 | 28.7 |
| Octyldodecyl myristate | | 16.8 | 16.2 | 54.0 | 15.2 | 50.7 | 17.2 | 57.3 |
| Propylene glycol monocaprylate | | 5.0 | 5.0 | 5.0 | 10.0 | 10.0 | 10.0 | 10.0 |
| Terpene resin | "PX1150N" YASUHARA CHEMICAL | 7.0 | 10.0 | 10.0 | 10.0 | 10.0 | 0 | 0 |

**Table 2**

| | | Comparative ex. 1 | Comparative ex. 2 | Comparative ex. 3 | Comparative ex. 4 | Comparative ex. 5 | Comparative ex. 6 |
|---|---|---|---|---|---|---|---|
| Tetracaine | | 4.0 | 4.0 | 4.0 | 4.0 | 4.0 | 4.0 |
| Styrene block copolymer | "Quintac 3520" Zeon Corporation | 23.1 | 20.3 | 24.6 | 71.7 | 68.8 | 28.7 |
| Octyldodecyl myristate | | 57.9 | 60.8 | 61.4 | 14.3 | 17.2 | 57.3 |
| Propylene glycol monocaprylate | | 5.0 | 5.0 | 10.0 | 10.0 | 0 | 0 |
| Terpene resin | "PX1150N" YASUHARA CHEMICAL | 10.0 | 10.0 | 0 | 0 | 10.0 | 10.0 |

### Test example 1: Evaluation of transdermal permeability

Hair was removed from the abdomen of 5-week-old male Wister rat, and the skin was obtained therefrom. The skin was placed on a vertical Franz diffusion cell (model number "TP-8s" manufactured by Vidrex). The patch prepared in Examples and Comparative examples was cut into a circular form having a diameter of 1.0 cm and adhered on the rat skin in the Franz diffusion cell. The test was conducted using 0.01 mol/L phosphate buffered saline (pH 7.2 to 7.4) having a temperature of 32°C as a buffer. A part of the buffer was taken as a sample after 3 hours from the start of the test, and an amount of the drug that had passed through the rat skin in the buffer was measured by HPLC. A 4% tetracaine gel preparation (trade name: "Ametop^{(R)} Gel") was used as a control preparation containing tetracaine and applied on the rat skin in the Franz diffusion cell. Then, the applied preparation was covered with polyvinylidene chloride film (trade name: "Saran Wrap (registered trademark) " manufactured by Asahi Kasei Home Products) so that the water in the gel was not volatilized. The test emulated occlusive therapy. The amount was measured three times per each patch, and an average value of the measurement values was calculated. The result was shown in Tables 3 and 4.

### Test example 2: Evaluation of cohesion force

The cohesion force of the adhesive layer of the patch was evaluated using finger tuck on the basis of the following four criteria. The result was shown in Tables 3 and 4.
3: adhesive residue was not observed at all.
2: adhesive residue was hardly observed, and there was no problem.
1: cohesive force was slightly insufficient but there was no problem.
0: adhesive residue and deformation were observed, and cohesive force was remarkably insufficient.

### Test example 3: Evaluation of adhesive force

The adhesive force of the adhesive layer of the patch was evaluated using finger tuck on the basis of the following four criteria. The result was shown in Tables 3 and 4.
3: higher adhesive force was shown in comparison with an already-existing local anesthetic patch ("Lidocaine tape YP" manufactured by YUTOKU PHARMACEUTICAL).
2: similar adhesive force was shown to an already-existing local anesthetic patch ("Lidocaine tape YP" manufactured by YUTOKU PHARMACEUTICAL) .
1: slightly lower adhesive force was shown in comparison with an already-existing local anesthetic patch ("Lidocaine tape YP" manufactured by YUTOKU PHARMACEUTICAL) containing gum adhesive backing layer.
0: the patch could not adhered and was remarkably peeled.

**Table 3**

| | Ex. 1 | Ex. 2 | Ex. 3 | Exl 4 | Ex. 5 | Ex. 6 | Ex. 7 |
|---|---|---|---|---|---|---|---|
| Higher fatty acid ester/ thermoplastic elastomer | 0.25 | 0.25 | 2.0 | 0.25 | 2.0 | 0.25 | 2.0 |
| Skin permeability of drug (mg/cm²) | 21.2 | 37.7 | 34.7 | 16.0 | 58.7 | 34.4 | 38.5 |
| Cohesion force | 3 | 3 | 2 | 3 | 2 | 3 | 1 |
| Adhesive force | 1 | 2 | 3 | 2 | 3 | 1 | 2 |

**Table 4**

| | Comparative ex. 1 | Comparative ex. 2 | Comparative exO 3 | Comparative ex. 4 | Comparative ex. 5 | Comparative ex. 6 |
|---|---|---|---|---|---|---|
| Higher fatty acid ester/ thermoplastic elastomer | 2.5 | 3.0 | 2.5 | 0.20 | 0.25 | 2.0 |
| Skin permeability of drug (mg/cm²) | - | - | - | - | 0.1 | 5.1 |
| Cohesion force | 0 | 0 | 0 | 3 | 3 | 3 |
| Adhesive force | - | - | - | 0 | 2 | 3 |

As the results shown in Tables 3 and 4, an adhesibility of the patches of Example 1 and Reference Example 6 was slightly low but was not a problem for actual use, and a cohesive force was good. A cohesive force of the patch of Reference Example 7 was slightly low but an adhesibility was good. All of the patches of Examples 2 to 5 showed an excellent cohesive force and adhesibility.

On the one hand, a ratio of a higher fatty acid ester in the patches of Comparative examples 1 to 3 to a thermoplastic elastomer was too large; as a result, an adhesibility could not be evaluated, since an adhesive layer could not be maintained due to a too low cohesive force. A ratio of a higher fatty acid ester of the patch of Comparative example 4 to a thermoplastic elastomer was too low; as a result, a cohesive force of the adhesive layer was observed but an adhesibility could not be observed. The patches of Comparative examples 5 and 6 had both of sufficient cohesive force and adhesibility but a transdermal permeability of the drug was not observed, since the patches did not contain a polyol fatty acid monoester.

In addition, Example 4 is compared with Comparative example 5 and Example 5 is compared with Comparative example 6, since ratios of a higher fatty acid ester to a thermoplastic elastomer of the Examples are the same as those of the Comparative examples; as a result, the transdermal permeability of Example 4 was very high as about 160 times to that of Comparative example 5 and the transdermal permeability of Example 5 was very high as about 11.5 times to that of Comparative example 6.

### Examples 9 to 12, 14 and 15, Reference Examples 8 and 13, Comparative examples 7 to 9: Production of patch

An amount of each component that constituted the adhesive layer was weighted in accordance with the compositions shown in Tables 5 and 6, and each patch was prepared similarly to the above-describe production method. In addition, a transdermal permeability, a cohesion force and an adhesibility were evaluated similarly to the above. The result is shown in Table 5. With respect to a skin permeability, a ratio to the transdermal permeability measurement value of a 4% tetracaine gel preparation (trade name: "Ametop^{(R)} Gel") as a control preparation is shown.

**Table 5**

| | | Ex. 8 | Ex. 9 | Ex. 10 | Ex. 11 | Ex. 12 | Ex. 13 | Ex. 14 | Ex. 15 |
|---|---|---|---|---|---|---|---|---|---|
| Tetracaine | | 5.3 | 5.3 | 5.5 | 5.5 | 5.3 | 5.3 | 5.3 | 5.3 |
| Styrene block copolymer | "Quintac 3520" Zeon Corporation | 35.0 | 35.0 | - | - | - | - | - | - |
| Styrene block copolymer | "JSR 5505" JSR | - | - | 35.0 | 35.0 | 35.0 | 35.0 | 31.0 | 35.0 |
| Octyldodecyl myristate | | 44.7 | 34.7 | 34.5 | 34.5 | 34.7 | 44.7 | 38.7 | 34.7 |
| 2-Hexyl-1-dodecanol | | - | - | - | - | - | - | - | - |
| Liquid paraffin | | - | - | - | - | - | - | - | - |
| Propylene glycol monocaprylate | | 10.0 | 10.0 | 15.0 | 10.0 | 10.0 | 10.0 | 10.0 | 10.0 |
| Propylene glycol dicaprylate | | - | - | - | - | - | - | - | - |
| Oleyl alcohol | | 5.0 | 3.0 | - | 5.0 | 5.0 | 5.0 | 5.0 | - |
| Lauryl alcohol | | - | - | - | - | - | - | - | 3.0 |
| Diisopropyl adipate | | - | 2.0 | - | - | - | - | - | 2.0 |
| Terpene resin | "PX1150N" YASUHARA CHEMICAL | - | 10.0 | 10.0 | 10.0 | 10.0 | - | 10.0 | 10.0 |
| Ratio of drug skin permeability to control preparation | | 2.69 | 3.82 | 2.20 | 2.28 | 2.95 | 3.48 | 2.40 | 3.40 |
| Cohesion force | | 3 | 3 | 3 | 2 | 3 | 3 | 3 | 3 |
| Adhesive force | | 3 | 3 | 3 | 3 | 3 | 1 | 2 | 1 |

**Table 6**

| | | Comparative ex. 7 | Comparative ex. 8 | Comparative exn 9 |
|---|---|---|---|---|
| Tetracaine | | 6.0 | 5.3 | 5.3 |
| Styrene block copolymer | "Quintac 3520" Zeon Corporation | 44.0 | 35.0 | 35.0 |
| Styrene block copolymer | "JSR 5505" JSR | - | - | - |
| Octyldodecyl myristate | | - | - | 34.7 |
| 2-Hexyl-1-dodecanol | | 22.0 | - | - |
| Liquid paraffin | | 15.0 | 34.7 | - |
| Propylene glycol monocaprylate | | 13.0 | 10.0 | - |
| Propylene glycol dicaprylate | | - | - | 10.0 |
| Oleyl alcohol | | - | 3.0 | 3.0 |
| Lauryl alcohol | | - | - | - |
| Diisopropyl adipate | | - | 2.0 | 2.0 |
| Terpene resin | "PX1150N" YASUHARA CHEMICAL | - | 10.0 | 10.0 |
| Ratio of drug skin permeability to control preparation | | 1.02 | - | 0.09 |
| Cohesion force | | 3 | 1 | 2 |
| Adherence property | | 1 | 0 | 3 |

As the results shown in Tables 5 and 6, any patches of Examples 8 to 15 had excellent cohesive force and adhesibility. On the one hand, the patch of Comparative example 8 did not show adhesibility at all, and transdermal permeability was not conducted, since oozing of the liquid component was observed.

A transdermal permeability of the patches of Examples 8 to 15 and Comparative examples 7 and 9 was evaluated with using a conventional tetracaine-containing gel preparation (trade name: "Ametop^{(R)} Gel") as a control preparation. As a result, a cumulative drug transdermal permeability amount from the patch of Comparative example 7 containing a higher alcohol and liquid paraffin in place of a higher fatty acid ester was similar to that of the gel preparation. The patch of Comparative example 9 that contained a higher fatty acid but that did not contain a polyol fatty acid monoester hardly showed a drug transdermal permeability. On the one hand, a cumulative drug transdermal permeability amount from the patches of Examples 8 to 15 were high as two or more times in comparison with that of the conventional gel preparation.

## Claims

1. A patch,
comprising a backing layer, and an adhesive layer on the backing layer,
wherein the adhesive layer comprises at least a local anesthetic agent, a thermoplastic elastomer, a higher fatty acid ester, and a polyol fatty acid monoester,
a ratio of the higher fatty acid ester to 100 mass parts of the thermoplastic elastomer is 25 mass parts or more and 200 mass parts or less,
a carbon number in an ester part of the higher fatty acid ester is 12 or more and 30 or less,
the higher fatty acid is capric acid or a fatty acid having a carbon number of 12 or more and 30 or less, and
the adhesive layer comprises 5 mass% or more of a tackifier.

2. The patch according to claim 1, wherein a ratio of the higher fatty acid ester to 100 mass parts of the thermoplastic elastomer is 30 mass parts or more and 150 mass parts or less.

3. The patch according to claim 1 or 2, wherein a content amount of the higher fatty acid ester in the adhesive layer is 60 mass% or less.

4. The patch according to any one of claims 1 to 3, wherein the thermoplastic elastomer is a styrene block copolymer.

5. The patch according to claim 4, wherein the styrene block copolymer is a mixture of a styrene-isoprene-styrene block copolymer and a styrene-isoprene block copolymer.

6. The patch according to claim 5, wherein a ratio of the styrene-isoprene block copolymer in the mixture is 50 mass% or more.

7. The patch according to any one of claims 4 to 6, wherein a viscosity of a 25 mass% toluene solution of the styrene block copolymer at 25°C is 500 mPa·s or more and 2000 mPa·s or less.

8. The patch according to any one of claims 1 to 7, wherein the polyol fatty acid monoester is a propylene glycol fatty acid monoester.

9. The patch according to any one of claims 1 to 8, wherein the adhesive layer further comprises a higher alcohol.

10. The patch according to claim 9, wherein a carbon number of the higher alcohol is 12 or more and 30 or less.

11. The patch according to claim 9 or 10, wherein the higher alcohol is oleyl alcohol and/or lauryl alcohol.

12. The patch according to any one of claims 1 to 11, wherein the local anesthetic agent is one or more local anesthetic agents selected from the group consisting of tetracaine, lidocaine, prilocaine, bupivacaine, mepivacaine, benzocaine, levobupivacaine and ropivacaine.

13. The patch according to any one of claims 1 to 11, wherein the local anesthetic agent is tetracaine.

## Patentansprüche

1. Pflaster,
umfassend eine Trägerschicht und eine Klebeschicht auf der Trägerschicht,
wobei die Klebeschicht mindestens ein Lokalanästhetikum, ein thermoplastisches Elastomer, einen höheren Fettsäureester und einen Polyolfettsäuremonoester umfasst,
ein Anteil des höheren Fettsäureesters bezogen auf 100 Masseteile des thermoplastischen Elastomers 25 Masseteile oder mehr und 200 Masseteile oder weniger beträgt,
eine Kohlenstoffzahl in einem Esterteil des höheren Fettsäureesters 12 oder mehr und 30 oder weniger beträgt,
die höhere Fettsäure Caprinsäure oder eine Fettsäure mit einer Kohlenstoffzahl von 12 oder mehr und 30 oder weniger ist und
die Klebeschicht 5 Masse-% oder mehr eines Klebrigmachers umfasst.

2. Pflaster gemäß Anspruch 1, wobei ein Anteil des höheren Fettsäureesters bezogen auf 100 Masseteile des thermoplastischen Elastomers 30 Masseteile oder mehr und 150 Masseteile oder weniger beträgt.

3. Pflaster gemäß Anspruch 1 oder 2, wobei eine Gehaltsmenge des höheren Fettsäureesters in der Klebeschicht 60 Masse-% oder weniger beträgt.

4. Pflaster gemäß einem der Ansprüche 1 bis 3, wobei das thermoplastische Elastomer ein Styrol-Blockcopolymer ist.

5. Pflaster gemäß Anspruch 4, wobei das Styrol-Blockcopolymer eine Mischung aus einem Styrol-Isopren-Styrol-Blockcopolymer und einem Styrol-Isopren-Blockcopolymer ist.

6. Pflaster gemäß Anspruch 5, wobei ein Anteil des Styrol-Isopren-Blockcopolymers in der Mischung 50 Masse-% oder mehr beträgt.

7. Pflaster gemäß einem der Ansprüche 4 bis 6, wobei eine Viskosität einer 25 masse-%igen Toluollösung des Styrol-Blockcopolymers bei 25°C 500 mPa·s oder mehr und 2.000 mPa·s oder weniger beträgt.

8. Pflaster gemäß einem der Ansprüche 1 bis 7, wobei der Polyolfettsäuremonoester ein Propylenglykolfettsäuremonoester ist.

9. Pflaster gemäß einem der Ansprüche 1 bis 8, wobei die Klebeschicht ferner einen höheren Alkohol umfasst.

10. Pflaster gemäß Anspruch 9, wobei eine Kohlenstoffzahl des höheren Alkohols 12 oder mehr und 30 oder weniger beträgt.

11. Pflaster gemäß Anspruch 9 oder 10, wobei der höhere Alkohol Oleylalkohol und/oder Laurylalkohol ist.

12. Pflaster gemäß einem der Ansprüche 1 bis 11, wobei das Lokalanästhetikum eines oder mehrere Lokalanästhetika, ausgewählt aus der Gruppe, bestehend aus Tetracain, Lidocain, Prilocain, Bupivacain, Mepivacain, Benzocain, Levobupivacain und Ropivacain, ist.

13. Pflaster gemäß einem der Ansprüche 1 bis 11, wobei das Lokalanästhetikum Tetracain ist.

## Revendications

1. Timbre transdermique,
comprenant une couche de support et une couche adhésive sur la couche de support,
dans lequel la couche adhésive comprend au moins un agent anesthésique local, un élastomère thermoplastique, un ester d'acide gras supérieur, et un monoester d'acide gras polyol,
un rapport de l'ester d'acide gras supérieur sur 100 parties en masse de l'élastomère thermoplastique est de 25 parties en masse ou plus et de 200 parties en masse ou moins,
un nombre d'atomes de carbone dans une partie ester de l'ester d'acide gras supérieur est de 12 ou plus et de 30 ou moins,
l'acide gras supérieur est l'acide caprique ou un acide gras présentant un nombre de carbones de 12 ou plus et de 30 ou moins, et
la couche adhésive comprend 5 % en masse ou plus d'un agent tackifiant.

2. Timbre transdermique selon la revendication 1, dans lequel un rapport de l'ester d'acide gras supérieur sur 100 parties en masse de l'élastomère thermoplastique est de 30 parties en masse ou plus et de 150 parties en masse ou moins.

3. Timbre transdermique selon la revendication 1 ou 2, dans lequel une teneur en ester d'acide gras supérieur dans la couche adhésive est inférieure ou égale à 60 % en masse.

4. Timbre transdermique selon l'une quelconque des revendications 1 à 3, dans lequel l'élastomère thermoplastique est un copolymère bloc styrène.

5. Timbre transdermique selon la revendication 4, dans lequel le copolymère bloc styrène est un mélange d'un copolymère bloc styrène-isoprène-styrène et d'un copolymère bloc styrène-isoprène.

6. Timbre transdermique selon la revendication 5, dans lequel un rapport du copolymère bloc styrène-isoprène dans le mélange est de 50 % en masse ou plus.

7. Timbre transdermique selon l'une quelconque des revendications 4 à 6, dans lequel une viscosité d'une solution à 25 % en masse de toluène du copolymère bloc styrène à 25 °C est de 500 mPa·s ou plus et de 2000 mPa·s ou moins.

8. Timbre transdermique selon l'une quelconque des revendications 1 à 7, dans lequel le monoester d'acide gras de polyol est un monoester d'acide gras de propylène glycol.

9. Timbre transdermique selon l'une quelconque des revendications 1 à 8, dans lequel la couche adhésive comprend en outre un alcool supérieur.

10. Timbre transdermique selon la revendication 9, dans lequel le nombre de carbones de l'alcool supérieur est de 12 ou plus et de 30 ou moins.

11. Timbre transdermique selon la revendication 9 ou 10, dans lequel l'alcool supérieur est l'alcool oléylique et/ou l'alcool laurique.

12. Timbre transdermique selon l'une quelconque des revendications 1 à 11, dans lequel l'agent anesthésique local est un ou plusieurs agents anesthésiques locaux sélectionnés parmi le groupe consistant en la tétracaïne, la lidocaïne, la prilocaïne, la bupivacaïne, la mépivacaïne, la benzocaïne, la lévobupivacaïne et la ropivacaïne.

13. Timbre transdermique selon l'une quelconque des revendications 1 à 11, dans lequel l'agent anesthésique local est la tétracaïne.
